# EUROPEAN PATENT APPLICATION

(11) **EP 2 875 834 A1**
(43) Date of publication of application: **27.05.2015**
(21) Application number: 14194563.4
(22) Date of filing: 24.11.2014
(51) Int. Cl.: A61L 31/10, A61L 31/14, A61L 31/16, A61L 17/10, A61L 17/14

(54) **Medical devices and methods of manufacture thereof**

(30) Priority: 26.11.2013 US 201361909019 P
(71) Applicant: Cook Medical Technologies LLC, Bloomington, IN 47404 (US)
(72) Inventor: Albanna, Mohammad Z., Winston-Salem, NC 27104 (US)
(74) Representative: Garratt, Peter Douglas

(57) **Abstract**

The embodiments relate generally to medical devices and to methods of their manufacture. One aspect provides devices including chitosan fibers that are a free of chemical cross linking. Another aspect provides a method of manufacturing such devices.

## Description

### RELATED APPLICATIONS

The present patent application claims the benefit of the filing date under 35 U.S.C. §119(e) of U.S. Provisional Patent Application Serial No. 61/909,019, filed November 26, 2013.

### TECHNICAL FIELD

The embodiments relate generally to medical devices and to methods of their manufacture. In certain embodiments, the devices are braided or woven devices that are partly or completely implantable into a human or veterinary patient.

### BACKGROUND

A variety of treatment and diagnostic procedures include the step of implanting a device into the body of a patient. Such devices include stents, for example, vascular, urinary or biliary stents. Stents can be made both of metal and polymeric materials. Polymeric stents, particularly bioabsorbable polymer stents, offer advantages including in situations when the stent is not intended for permanent placement. In such applications, a device that is absorbed after a certain time in the body eliminates the need for a separate procedure to remove the device.

The use of bioabsorbable devices can also prevent injury to the patient. For example, if a non-absorbable device is left within the vascular system for an extended period of time, a thrombus sometimes forms on the device itself, causing stenosis or occlusion. As a result, the patient is placed at risk of a variety of complications, including heart attack, pulmonary embolism and stroke. In addition, permanent devices, particularly metal devices, often interfere with subsequent diagnostic imaging evaluations.

However, the use of such polymeric devices may introduce additional problems, such as premature absorption of the device structure resulting in collapse and blockage of a vessel. The use of polymeric devices may also be constrained due to their limited strength, for a given thickness, and their inability to resist external compression forces. In the case of bioabsorbable polymeric devices, inability to control the degradation of the device presents additional problems. A major limitation of conventional biodegradable devices, particularly stents, can occur due to the sudden breakdown of the device into large fragments, which can obstruct fluid flow within a body lumen.

For example, ureteral stenting serves as a minimally invasive approach to prevent ureter occlusion by kidney stones and preserve urine drainage. The ideal ureteral stent should be well tolerated by the patient, biocompatible, visible on the ultrasound (or other compatible imaging modality), easily inserted and removed, resistant to infection and corrosion or oxidation, and provide seamless urine flow. Despite the substantial technical improvements over the last 30 years in stent designs and composition of materials, conventional stents have not yet met all of the desirable features for an ideal ureteral stent.

### SUMMARY

One aspect of the present invention provides a medical device including a plurality of chitosan fibers. In one embodiment, the chitosan fibers are free of a chemical cross linking agent and can form a braided structure, such as a radially expandable tubular structure. In another embodiment the medical device is a bioabsorbable medical device. The medical device can be, for example, a catheter, a stent, a urinary stent, a biliary stent, a suture or a woven mat. In certain embodiments the device includes a bioactive agent on a surface, or within, at least one of the fibers.

In another aspect, the present invention provides a method of manufacturing a medical device including a plurality of fibers. In certain embodiments, the method includes extruding a composition containing chitosan into a coagulation bath to form elongated chitosan fibers and drying the fibers. The coagulation bath can contain a basic solution. The fibers and woven or braided to form all, or part, of a medical device. In certain embodiments, the fibers are not cross linked with a chemical cross-linking agent.

The composition can be a solution of chitosan in acetic acid and the basic solution can be an ammonia solution. In certain embodiments, the fibers are dried, preferably under tension, at a temperature of between 90 degrees C and 200 degrees C.

### DETAILED DESCRIPTION OF THE PREFERRED EMBODIMENTS

### Definitions

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention. All publications, patent applications, patents and other references mentioned herein are incorporated by reference in their entirety.

The uses of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example") provided herein, is intended merely to better illuminate the invention and does not pose a limitation on the scope of the invention unless otherwise claimed. No language in the specification should be construed as indicating any non-claimed element as essential to the practice of the invention.

As used herein the terms "comprise(s)," "include(s)," "having," "has," "can," "contain(s)," and variants thereof, are intended to be openended transitional phrases, terms, or words that do not preclude the possibility of additional acts or structures. The present invention also contemplates other embodiments "comprising," "consisting of" and "consisting essentially of," the embodiments or elements presented herein, whether explicitly set forth or not.

As used herein, the term "implantable" refers to an ability of a medical device to be positioned at a location within a body, such as within a body vessel. Furthermore, the terms "implantation" and "implanted" refer to the positioning of a medical device at a location within, or partially within, a body, such as within a body vessel.

The term "bioabsorbable" is used herein to refer to materials that dissipate upon implantation within a body, independent of which mechanisms by which dissipation can occur, such as dissolution, degradation, absorption and excretion.

As used herein, the term "vessel" means any body passage or lumen, including but not limited to blood coronary or peripheral vessels, esophageal, intestinal, biliary, urethral and ureteral passages.

As used herein, the term "bioactive" refers to any agent that produces an intended therapeutic effect on the body to treat or prevent conditions or diseases.

A "therapeutically-effective amount" as used herein is the minimal amount of a bioactive which is necessary to impart therapeutic benefit to a human or veterinary patient.

### Implantable Devices

For the purpose of promoting an understanding of the principles of the invention, reference will now be made to embodiments, and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates.

One aspect of the present invention provides an implantable medical device including a base structure having, at least, a bioadsorbable portion. In various embodiments, the bioabsorbable portion is formed from a bioabsorbable polymer. A wide variety of bioabsorbable polymers can be used to form the device structure. Nonlimiting examples of bioabsorbable polymers include polyesters such as poly(hydroxyalkanoates), poly(lactic acid) or polylactide (PLA), poly(glycolic acid) or polyglycolide (PGA), poly(caprolactone), poly(valerolactone) and co-polymers thereof; polycarbonates; polyoxaesters such as poly(ethylene oxalate), poly(alkylene oxalates); polyanhydrides; poly(amino acids); polyphosphazenes; phosphorylcholine; phosphatidylcholine; various hydrogels; polydioxanone, poly(DTE carbonate), and co-polymers or mixtures of two or more of the above polymers. The implantable devices can also include various natural polymers such as fibrin, collagens, extracellular matrix (ECM) materials, dextrans, polysaccharides, chitosan and hyaluronic acid.

By way of example, the medical device can be or include a catheter, a stent, a stent graft, a coil, a needle, a suture, a graft, a filter, a scaffold or any combination of these. The catheter may be, for example, a urethral catheter, a catheter for nephrostomy drainage, a catheter for nasal pancreatic drainage, a catheter for suprapubic drainage, or a nasal biliary drainage catheter. The stent may be, for example, a coronary or peripheral vascular stent, a urethral stent, a prostatic stent, a biliary stent or a pancreatic stent.

In one embodiment, the device is a vascular stent. Such stents are typically about 10 to about 60 mm in length and designed to expand to a diameter of about 2 mm to about 6 mm when inserted into the vascular system of the patient. These stent dimensions are, of course, applicable to exemplary stents employed in the coronary arteries. Structures such as stents or catheter portions intended to be employed at other sites in the patient, such as in the aorta, peripheral vascular system, esophagus, trachea, colon, biliary tract, or urinary tract will have different dimensions more suited to such use. For example, aortic, esophageal, tracheal and colonic stents may have diameters up to about 25 mm and lengths about 100 mm or longer.

In another embodiment, the device is a braided stent sized and shaped to be anchored either at the calyx of the kidney, an opening of the ureter in the bladder, or at both ends for firm fixation and prevention of stent migration. The braided stent can be fabricated with varying thickness (i.e. diameter) ranging, for example, from 6-8 Fr and length ranging from 20-30 cm to accommodate individual patient needs. Examples of different designs of a stent to be deployed in the ureter include (1) braided stents with pig-tail hooks at each end; (2) a braided fibrous mat rolled over to form a tubular construct; (3) braided fibrous bundles positioned next to each other around thin tubular membrane; (4) braided fibrous bundles positioned next to each other to cover the lumen of a tubular thin membrane; (5) braided fibrous bundles processed to make a woven mesh then rolled to form a tubular construct; or (6) braided fibrous bundles positioned next to each other in a circular orientation and attached to each other through a dehydrated hydrogel to form a tubular construct.

In certain embodiments, the devices have at least a portion that is configured to expand during deployment so as to contact walls of the vessel in which they are implanted to anchor the device within the vessel. In this regard, both self-expanding and force-expandable (e.g. balloon-expandable) stents or other implantable medical devices are contemplated as being within the scope of embodiments of the present invention. It is also contemplated that the device may be configured for introduction by a minimally-invasive surgical technique, especially percutaneous introduction, or may be configured for introduction by invasive surgery in which the site of intended implantation in the body vessel or other site is surgically exposed from the exterior of the patient for introduction of the implantable device. The implantable device may also be percutaneously retrievable, for example a percutaneously retrievable stent, filter or frame. These and other variations in the implantable device and its associated procedure for introduction will be apparent to those skilled in the pertinent art from the descriptions herein.

In one embodiment, the device includes a braided, or woven, structure formed from fibers of chitosan, a linear polysaccharide composed of randomly distributed β-(1-4)-linked D-glucosamine (deacetylated unit) and N-acetyl-D-glucosamine (acetylated unit). In certain embodiments, the chitosan fibers are not modified or cross linked with a chemical cross linking agent, i.e. the chitosan fibers are free of chemical cross linking.

In certain embodiments, the device includes chitosan fibers having the fiber diameter between 100 and 500, 100 and 400, 100 and 300, 100 and 200, 500 and 400, 500 and 300 or 500 and 200 microns. In other embodiments, the fibers contain a crystalline chitosan microstructure. For example, the fibers can include at least 20, 40, 50, 60, 70, 80, 90 or more percentage of a crystalline chitosan form.

In certain embodiments, the device includes chitosan fibers that are woven or braided to form at least part of the structure of the device. In some embodiments, the device includes a braided or woven structure including fiber bundles containing 4, 5, 6, 7, 8, 9, 10 or more fibers. The device can also include a structure including fibers within the bundles that are braided or otherwise twisted to form the bundle structure. The individual bundles may be combined, again by braiding or otherwise twisting, to form the structure of the device.

In certain embodiments, the mechanical properties, including the degradation rate, of the device are varied by controlling the number of fibers in each bundle, number of bundles, and the degree of twisting and twisting direction, both within the bundles and between the bundles. The degree of twisting and twisting direction affects the device flexibility, strength, and level of abrasion resistance. Increasing the amount of twist increases the device strength and improves the level of abrasion resistance of the device.

### Bioactive Coated Devices

In certain embodiments, the implantable device includes a therapeutically-effective amount of a bioactive that either elutes from the device for implantation into the patient or is bonded to the device in a permanent manner. For example, the fibers can be coated with a bioactive, such as heparin or another thrombin inhibitor; hirudin or another antithrombogenic agent; urokinase or another thrombolytic agent; a fibrinolytic agent; a vasospasm inhibitor; a calcium channel blocker; nitric or another vasodilator; terazosin or another antihypertensive agent; an antimicrobial agent; an antibiotic; an antiplatelet agent; an antimitotic; a microtubule inhibitor; an actin inhibitor; a remodeling inhibitor; deoxyribonucleic acid; an antisense polynucleotide; methotrexate or another antiproliferative agent; tamoxifen citrate; a taxane agent, such as paclitaxel or a derivative thereof; a mammalian target of rapamycin (mTOR) inhibitor such as sirolimus or a derivative thereof such as pimecrolimus, tacrolimus, everolimus, zotarolimus, novolimus, myolimus, temsirolimus, deforolimus, or biolimus; an anti-cancer agent; dexamethasone or a dexamethasone derivative; an anti-inflammatory steroid or non-steroidal antiinflammatory agent; cyclosporin or another immunosuppressive agent; a peptide; a protein; an enzyme; an extracellular matrix component; a cellular component or another biologic agent; captopril; enalapril or another angiotensin converting enzyme (ACE) inhibitor; ascorbic acid; alpha tocopherol; superoxide dismutase; deferoxamine; an iron chelator or antioxidant; or combinations or mixtures of at least two of these agents.

The bioactive containing layer can include a carrier material. For example, the bioactive can be present in a layer also including one or more bioabsorbable polymers. In these embodiments, the polymer can exhibit properties that differ from those of the underlying structure. For example, the polymer can have a different absorption profile upon implantation. In other embodiments, the coating layer does not include a carrier material, such as a polymer. For example, the coating may include only the bioactive or the bioactive and other components that do not affect the elution of the bioactive. For the purposes of describing the present embodiments, the bioactive containing layer is considered to "consist essentially" of the bioactive or bioactives when it is free of materials, such as carrier materials, that affect the elution rate of the bioactive upon implantation.

In other embodiments, at least one bioactive is contained within the structure of the device, for example, within the fibers forming the device. For example, the bioactive can be included in a mixture extruded to form at least part of the structure of the device. In other embodiments, the bioactive is imbibed into the fibers forming the device. The present embodiments also include those having the same, or different, bioactives are present within the fiber structure and as a coating on the fibers.

### Methods of Manufacture

Another aspect of the present invention provides a method of manufacturing a device including a structure formed from a polymer, such as chitosan. In various embodiments, chitosan fibers are formed by an extrusion or wet spinning technique and then braided or woven to form the structure.

In the polymer fiber extrusion technique, a polymeric solution is extruded through small-diameter nozzle directly into a coagulation bath to form a hydrogel fiber. After neutralization in the bath, the fibers are dried, preferably under tension. The dried fibers are then collected and used to produce various three dimensional structures such as braided tubes, sutures, mats, fiber-reinforced, woven and braided constructs. The fibers can be produced with varying diameters and mechanical properties without adversely affecting fiber biocompatibility. In one embodiment, the fiber diameter and the mechanical properties of the fiber are altered by selectively changing parameters such as the polymer concentration, extrusion and coagulation bath buffer pH and type, and drying temperature.

Previous efforts have improved the mechanical properties of chitosan fibers through chemical modifications, including chemical cross-linking. For example, chemical cross-linking agents, such as epichlorhydrin, glutaraldehyde or sulphuric acid, have been used to modify the mechanical and biological properties of chitosan fibers. However, such modifications have resulted in increased toxicity, which has limited the use of devices containing chitosan, including for tissue engineering applications.

In certain embodiments, the present manufacturing process does not include the exposure of the fibers to a chemical cross-linking agent. Instead, the manipulation of the aforementioned parameters, for example polymer concentration, extrusion and coagulation bath buffer pH and type, and drying temperature, changes the fiber microstructure and results in substantial modification of fiber physical properties, including the fiber degradation rate.

The mechanical properties and degradation rate of a braided or woven device, such as a stent, can also be varied by controlling parameters such as the number of fibers in each fiber bundle, the number of fiber bundles, and the degree of bundle twisting and twisting direction. Particularly, degree of twisting and twisting direction significantly affect yarn flexibility, strength, and level of abrasion resistance. Increasing the amount of twist increases the yarn strength and improves the level of abrasion resistance of the yarns.

In various embodiments, chitosan fibers are formed by extruding a solution containing chitosan into a basic solution and then drying the resulting fibers at an elevated temperature. In one embodiment, chitosan is extruded in a solution containing at least 1, 2, 3, 4, 5, 6, or more percentage weight of acetic acid or between 1 and 2, 1 and 4, 1 and 6, 2 and 6, 4 and 6 percentage weight of acetic acid. In another embodiment, the coagulation bath contains at least 5, 10, 15, 20, 25, 30, 35 or more percentage of ammonia or between 5 and 35, 10 and 35, 15 and 35, 20 and 35, 25 and 35, 30 and 35, 5 and 30, 5 and 25, 5 and 20, 5 and 15, or 5 and 10 percentage of ammonia. In yet another embodiment, the fibers are dried under tension of a temperature of between 25 and 200, 40 and 200, 60 and 200, 90 and 200, 150 and 200, 25 and 40, 25 and 60, 25 and 90 or 25 and 150 deg. C. One aspect of the present invention provides chitosan fibers with improved mechanical and biocompatibility properties. The structure-property relationships of extruded chitosan fibers were explored by varying

### Examples

### Example 1 - Preparation of Chitosan Fibers

High molecular weight chitosan from crab shells (MW of 450 KDa, 75-85% degree of deacetylation), and 1-ethy1-3-(3-dimethylaminopropyl)-carbodiimide (EDC) are available from Sigma-Aldrich (St. Louis, MO). Heparin sodium USP (average molecular weight 10-12 KDa) is available from Celsus Laboratories (Cincinnati, OH). All other chemicals and solvents are of analytical reagent grade.

Chitosan powder is dissolved in different concentrations of acetic acid (1, 2, 3 and 6 % vol) to form 1.5 % wt chitosan solutions. Chitosan fibers are formed by extruding chitosan solutions through a 26 gauge Teflon catheter directly into either 10 % wt% or 25 % wt ammonia solution. For all experiments, chitosan solution is extruded at a constant rate of 85 ml min-1 using a syringe pump. Fibers are annealed by drying under tension in a lab oven at temperatures of 25, 40, 90, 140, and 195 °C, for 15 min and then cooled down slowly at a rate of 1 °C min⁻¹.

### Example 2 - Measurement of Fiber Diameter

Fibers, prepared as described above, are fully rehydrated in PBS. Phase contrast microscopy images of wet fibers are captured using a Nikon DIAPHOT 300 microscope. Fiber diameters are measured using the Sigma Scan Pro image analysis software (SPSS Inc., Chicago, IL).

### Example 3 - Evaluation of Fiber Mechanical Properties

After rehydration, mechanical properties of individual fibers are determined in the wet state using uniaxial tensile testing on a MTS Bionix 100 testing machine, at a constant strain rate of 1 min⁻¹. Tensile strength, breaking strain and modulus of elasticity at 2% strain are evaluated for all samples and average values (n =20/condition) are determined.

### Example 4 - Effect of Extrusion Buffer on Fiber Properties

Fibers are extruded using a solution of acetic acid. Increasing AA concentration is hypothesized to alter chitosan molecular architecture in solution by increasing the degree of protonation and hence the molecular extension. The resulting increased chain linearity due to intra-molecular repulsion is hypothesized to produce greater organization or crystallinity in the final material. Increasing AA concentration increases fiber diameters. An approximate 30% increase in fiber diameter at 6% acetic acid, compared to 1% acetic acid.

Fibers tensile strength shows a 70% improvement in fiber strength (from 0.7 to 1.2 MPa) when acetic acid concentration is increased from 1 to 2 % vol%. Above 2 % vol, fiber strength decreases steadily to 0.3 MPa at 6 % vol. Increasing acetic acid concentration from 1 % vol to 6 % vol results in decreasing fiber elasticity. Fiber stiffness improves by 2-fold from 3.5 to 7 MPa as the acetic acid concentration increases from 1 % vol to 3 % vol and then decreases to 3 MPa at 6 % vol.

### Example 5 - Effect of Coagulation Bath Buffer on Fiber Properties

A coagulation bath, containing strong base, for example ammonia, is used to produce chitosan polymer fibers. Increasing the pH of the coagulation bath is hypothesized to accelerate the neutralization of chitosan fibers and thus increase the formation of crystalline microstructure and reduce fiber swelling upon extrusion. To validate this hypothesis, the concentration of ammonia in the coagulation bath is increased from 10 % wt up to 25 % wt. Results show that when chitosan solution is extruded in 25 % wt ammonia solution, fiber diameters significantly decrease from 352 to 286 microns. This reduction in fiber diameter suggests reduced swelling due to the formation of crystalline microstructure.

The formation of crystalline microstructure is confirmed by XRD studies. XRD spectrum of wet chitosan fibers shows two dissimilar peaks. The first peak is a sharp and intense peak at 2θ=21° represents the crystalline fraction, while the second peak at 2θ=27° is broader and less intense than the first peak and represents the formation of an amorphous fraction. The crystalline peak at 2θ=21° was more prominent in fibers extruded in the 25 % wt ammonia solution. The ratio of crystalline to amorphous peak heights, 2θ₂₁o/2θ₂₇o is higher for fibers extruded in 25 % wt ammonia concentration confirming that fibers extruded in higher ammonia concentration have more crystalline fractions in their microstructure.

Fiber strength improves from 0.7 to 1.2 MPa when 25 % wt ammonia solution is used to neutralize the fibers. Fiber elasticity decreases by 2-fold from 0.2 to 0.09 when chitosan is extruded in 25 % wt ammonia solution. Extruding chitosan solution in higher ammonia concentration results in a 4-fold improvement in fiber stiffness from 3.5 to 13 MPa. Mechanical testing results confirm the proposed hypothesis that higher ammonia concentration results in rapid fiber neutralization and formation of predominant crystalline fraction in fiber microstructure.

### Example 6 - Effect of Drying Temperature on Fiber Properties

Drying fibers under tension at high temperature is known to cause significant changes in the microstructure of fibers. Annealing of chitosan fibers at an elevated temperature is hypothesized to improve their mechanical properties due to the rapid formation of crystalline microstructure. Different temperatures are studied as drying temperatures and results are compared to fibers dried at room temperature.

A change in fiber color from white to gold is observed when chitosan fibers dried at temperature higher than 25°C. Measurement of fiber diameters reveals a reduction in fiber diameters as temperature increases. The reduction in fiber diameters is significant at temperatures higher that 90°C suggesting major changes in fiber microstructure.

Testing of fiber mechanical properties shows that fiber strength improves as annealing temperature increases. Significant 2-fold and 3-fold improvements in fiber strength are observed at 140°C and 195°C respectively. Fiber elasticity slightly improves when temperature increases up to 90°C and then decreases thereafter. Fiber stiffness continues to increase with increasing temperatures. Increase in fiber stiffness is more significant after 90°C with an 8-fold improvement at 195°C.

XRD spectra of chitosan fibers annealed at different temperatures have three distinct peaks. Amorphous peaks were present at 2θ=9° and 27° and crystalline peak at 2θ=21°. Compared to fibers dried at higher temperatures, chitosan fibers that are dried at room temperature have a broader and less sharp crystalline peak. Also, the second amorphous peak (2θ=27°) is much broader compared to the same peak in different annealing temperatures. This suggests a less organized microstructure and lower mechanical properties which is confirmed by the mechanical testing results.

The peak height ratio increases as the annealing temperature increases indicating that the microstructure is shifting toward the crystalline structure. The increase in the crystalline structure causes an improvement in mechanical properties. This was confirmed by the improvement in mechanical properties with increasing the temperature.

Although the invention has been described and illustrated with reference to specific illustrative embodiments thereof, it is not intended that the invention be limited to those illustrative embodiments. Those skilled in the art will recognize that variations and modifications can be made without departing from the true scope and spirit of the invention as defined by the claims that follow. It is therefore intended to include within the invention all such variations and modifications as fall within the scope of the appended claims and equivalents thereof.

## Claims

1. A medical device comprising:
a plurality of chitosan fibers,
wherein the plurality of chitosan fibers are free of chemical cross linking.

2. The medical device of claim 1, wherein the plurality of chitosan fibers form a braided structure, preferably wherein the braided structure is a radially expandable tubular structure.

3. The medical device of any one of the preceding claims, wherein the medical device is a bioabsorbable medical device.

4. The medical device of any one of the preceding claims, wherein the device is selected from the group consisting of a stent, a urinary stent, a biliary stent, a suture, and a woven mat.

5. The medical device of any one of the preceding claims, further comprising a coating comprising a bioactive agent on a surface of the plurality of fibers, or further comprising a bioactive contained within at least one of the plurality of fibers.

6. The medical device of any one of the preceding claims, wherein the plurality of chitosan fibers comprises a crystalline microstructure.

7. The medical device of any one of the preceding claims, wherein at least one of the plurality of chitosan fibers is of a diameter between 100 and 500 microns.

8. A method of manufacturing a medical device, comprising
extruding a composition comprising chitosan to form a plurality of elongated fibers,
collecting the plurality of elongated fibers in a basic solution,
drying the plurality of elongated fibers under tension,
placing at least three of the plurality of fibers side by side to form an elongated strand, and
braiding the elongated strand to form a braided structure;
wherein the extruding and the drying are performed in the absence of a chemical crosslinking agent.

9. The method of claim 8, wherein the composition comprising chitosan further comprises acetic acid at a concentration of between 1 percentage weight and 6 percentage weight.

10. The method of claim 8 or claim 9, wherein the basic solution comprises a 10 to 30 percentage weight ammonia solution.

11. The method of any one of claim 8 to claim 10, wherein drying of the plurality of fibers is performed at a temperature of between 90 degrees C and 200 degrees C.

12. The method of any one of claim 8 to claim 11, further comprising coating a surface of the plurality of fibers with a bioactive agent, preferably wherein the bioactive agent comprises heparin.

13. The method of any one of claim 8 to claim 12, wherein the composition comprising chitosan further comprises a bioactive agent.

14. The method of any one of claim 8 to claim 10, wherein the braided structure is selected from the group consisting of a stent, a urinary stent, a biliary stent, a suture, and a woven mat.

15. The method of any one of claim 8 to claim 14, wherein the braided structure is a radially expandable tubular structure.
